# EUROPEAN PATENT APPLICATION

(11) **EP 1 371 387 A1**
(43) Date of publication of application: **17.12.2003**
(21) Application number: 03253727.6
(22) Date of filing: 12.06.2003
(51) Int. Cl.: A61M 35/00, A61F 13/00, A61F 13/38

(54) **Applicator and application method**

(30) Priority: 13.06.2002 JP 2002172337
(71) Applicant: Sanyo Co., Ltd, Tondabayashi-shi, Osaka 584-0022 (JP)
(72) Inventor: Nakatani, Hiroshi, Tondabayashi-shi, Osaka 584-0001 (JP)
(74) Representative: W.P. Thompson & Co.

(57) **Abstract**

An applicator includes a shank, an application liquid-impregnated part disposed on at least one end of the shank, and a film covering the shank and application liquid impregnated part. The film comprises a shank cover region and an impregnated part cover region that is formed integrally with and separable from the shank cover region. The shank cover region of the film is formed in such a small width that the shank can be gripped in the state the shank is covered with said film. Therefore, it is avoidable that the user's hand becomes dirty with an application liquid such as a medical liquid by separating only the impregnated part cover region and then gripping the shank through the film covering the shank.

## Description

### FIELD OF THE INVENTION

The present invention relates to an applicator for applying a medical liquid used for medical care such as disinfection, sterilization and medical treatment, makeup and cleaning of machine parts, electronic parts or the like. The invention also relates to an application method.

### BACKGROUND OF THE INVENTION

It has been conventional practice to wind absorbent cotton around one end of a shank, impregnate a medical liquid such as disinfectant in the absorbent cotton, and apply this to an external wound or an affected part such as the throat. It is however tedious to wind absorbent cotton and impregnate the medical liquid therein. In order to avoid this, it has been proposed to attach a fiber aggregate such as absorbent cotton to one end of a shank, a medical liquid is then impregnated in the fiber aggregate, and the resultant is sealingly packed individually with a gas impervious film. With the use of this applicator, the medical liquid can directly be applied to the affected part by unpacking the package and pinching the shank with the fingers.

However, this applicator suffers from the problem that a medical liquid is liable to seep from the fiber aggregate due to temperature changes or when pressure is applied from the exterior during post-manufacture storage or transportation. This results in the possibility that the seeped medical liquid adheres on the surface of the shank and the hand becomes dirty when taking out the shank.

In order to solve this problem, it has been proposed to pack only an application liquid-impregnated part (Japanese Patent Unexamined Publication Nos. 11-47191 and 2000-237235). However, by packing only the application liquid impregnated part, it is difficult to maintain gas tightness for a long period of time, and there is also the possibility that the application liquid leaks through the shank to the exterior.

### SUMMARY OF THE INVENTION

A main object of the present invention is to provide an applicator and application method, in which an application liquid such as a medical liquid is previously impregnated in an application liquid impregnated part, and which precludes the possibility that the hand becomes dirty with the application liquid at the time of use.

Another object of the present invention is to provide an applicator and application method, with which the application of a medical liquid or the like can be performed easily and sanitarily.

In order to solve the above problem, an applicator of the present invention includes a shank, an application liquid-impregnated part provided on at least one end of the shank, and a film covering the shank and application liquid-impregnated part. The film comprises a shank cover region and an impregnated part cover region that is formed integrally with and separable from the shank cover region. The shank cover region of the film is formed in such a small width that the shank can be gripped in the state the shank is covered with said film. The film is preferably a gas impervious film.

An application method using this applicator comprises steps of exposing an application liquid-impregnated part by separating an impregnated part cover region from a shank cover region in the film, and then performing application while gripping the shank covered with the film.

It is therefore possible to apply an application liquid such as a medical liquid easily and sanitarily, without making the hand dirty with the application liquid. In addition, since a certain amount of application liquid is previously impregnated in the application liquid impregnated part, it is possible to prevent disadvantages such as drips caused by an excess of impregnated application liquid, and an insufficient amount of application liquid.

The term "region covering the application liquid-impregnated part" (impregnated part cover region) in the present invention is to include not only a region directly covering an application liquid impregnated part, but also locations covering part of a shank adjacent to the application liquid impregnated part, as long as they constitute no obstruction to grip of the shank via the film.

By way of example only, specific embodiments on the present invention will now be described, with reference to the accompanying drawings, in which:-
Fig. 1 is a front elevation, partially broken away, of an applicator according to one preferred embodiment of the present invention;
Figs. 2(a) and 2(b) are partially enlarged views of notch parts;
Fig. 3 is a front view of an applicator employing perforated lines as separating means;
Fig. 4 is a sectional view of a gas impervious film employing perforated lines as separating means;
Figs. 5(a) and 5(b) are diagrams to explain the use of an applicator of the present invention;
Fig. 6 is a perspective view of the applicator as being used;
Fig. 7 is a front elevation of an applicator according to other preferred embodiment of the present invention;
Fig. 8 is a diagram to explain the use of an applicator according to other preferred embodiment;
Fig. 9 is a front elevation of an applicator according to still other preferred embodiment of the present invention; and
Fig. 10 is a front elevation of an applicator according to still other preferred embodiment of the present invention.

### DETAILED DESCRIPTION OF THE INVENTION

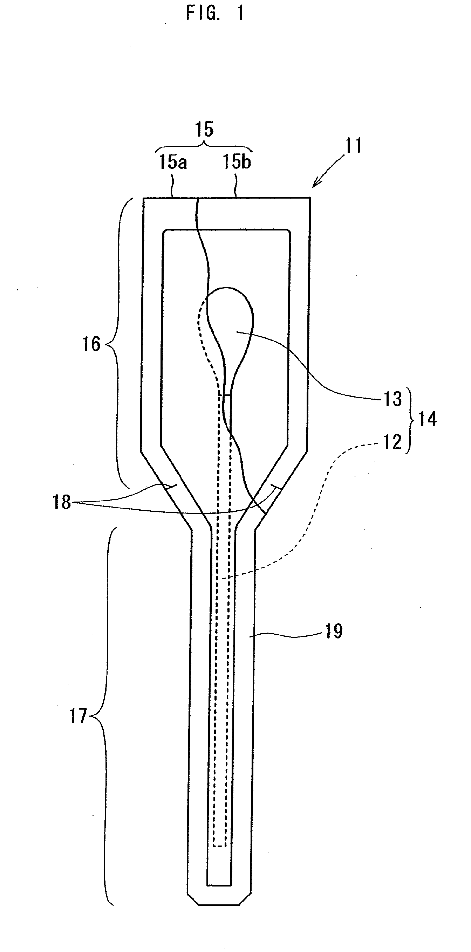

An applicator according to one preferred embodiment of the present invention will be described based on the accompanying drawings. Fig. 1 is a front elevation, partially broken away, of one preferred embodiment of the applicator in the present invention.

Referring to Fig. 1, an applicator 11 comprises a shank 12, an application liquid impregnated part 13 that is impregnated with an application liquid and is formed on one end of the shank 12, and a gas impervious film 15 for sealingly packing the shank 12 and application liquid-impregnated part 13. The gas impervious film 15 has on its peripheral part a sealing part 19. The sealing part 19 is provided with notch parts 18 (separating means) for separating an impregnated part cover region 16 covering the application liquid impregnated part 13 from the shank cover region 17 covering the shank 12.

As the shank 12, there can be used for example a plastic shank, paper shank, wood shank and metal shank. The thickness (outside diameter) of the shank 12 is not particularly limited. Preferably, the shank 12 is strong enough not to be broken during the use and has an easy-to-grip thickness during the use. The length of the shank 12 is also not particularly limited, and it can be set to any easy-to-use length.

As the application liquid-impregnated part 13, any material that can impregnate and apply an application liquid is usable. Examples are plastic foam and fiber aggregate. Alternatively, the part 13 may be one that is obtained by winding a non-woven fabric or woven fabric (e.g., gauze) around a shank. Examples of the fiber aggregate include a variety of natural fibers (cotton, silk, wool, etc.), regenerated fibers (rayon, cupra, etc.), and synthetic fibers (polyester fiber, polypropylene fiber, etc.). Most preferred is cotton fiber such as absorbent cotton. Such fiber is shaped in lump form such as an approximate sphere or ellipse, and is, as required, fixed to the shank 12 with adhesive.

The thickness (outer diameter) and length of the application liquid impregnated part 13 are not particularly limited but preferably the part 13 is of a size to impregnate sufficiently the necessary amount of application liquid. In the following description, for the sake of convenience, the shank 12 having the impregnated part 13 is referred to as a "swab 14" in some cases.

As the gas impervious film 15, any one that can prevent volatilization of an application liquid for a long period of time is usable. One example is that obtained by laminating a film for thermal bonding such as polyethylene film on metallic foil such as aluminum foil. In order to sealingly pack the swab 14 with the gas impervious film 15, gas impervious films 15a and 15b are disposed on upper and lower sides of the applicator 14 such that their respective films for thermal bond are opposed to each other, and the sealing part 19 disposed on the peripheral part is heated and pressed to obtain a thermal bond, so that the periphery is subjected to heat sealing, thereby obtaining the applicator 11 that sealingly houses the swab 14 in the gas impervious film 15. Although the swab 14 is sealingly packed with the film for thermal bond in this preferred embodiment, it may be sealed by applying adhesive to the peripheral part, instead of the film for thermal bond.

Although the shape of the gas impervious film 15 is not particularly limited, its width is preferably changed so as to correspond to the thickness of the shank 12 and application liquid impregnated part 13, as shown in Fig. 1. In particular, the shank cover region 17 is preferably sufficiently narrow to house the shank 12. This facilitates gripping of the shank 12 via the gas impervious film 15 at the time of use.

Further, the plastic film, such as the above-mentioned film for thermal bond, in the gas impervious film 15 is preferably a uniaxial oriented film that facilitates unpacking.

The sealing width of the sealing part 19 is not particularly limited and is determined in consideration of the volatility of an application liquid to be impregnated in the application liquid impregnated part 13.

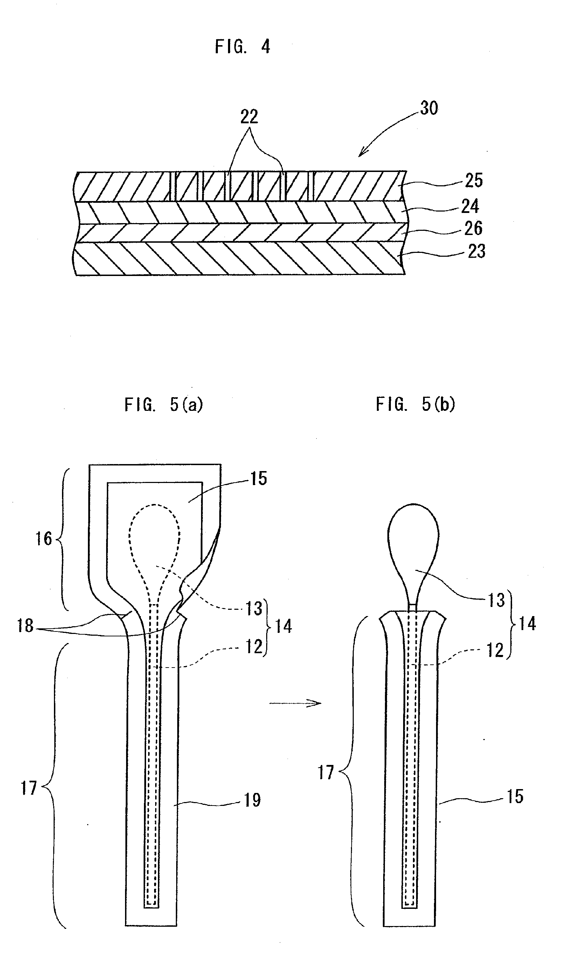

The notch part 18 is provided at a portion of the sealing part 19 located between the impregnated part cover region 16 and shank cover region 17. As shown in Fig. 1, the notch part 18 is obtained by forming a linear slit that extends obliquely from the edge of the sealing part 19 to the impregnated part cover region 16. The notch part 18 might be provided at only one end portion of the sealing part 19. In this case, the gas impervious film 15 cleaves in its width direction because of the uniaxial oriented film. Therefore, the unpacked shank cover region 17 is chamfered at its end part as shown in Fig. 5(b). This prevents a sharp point from erroneously abutting the affected part at the time of application.

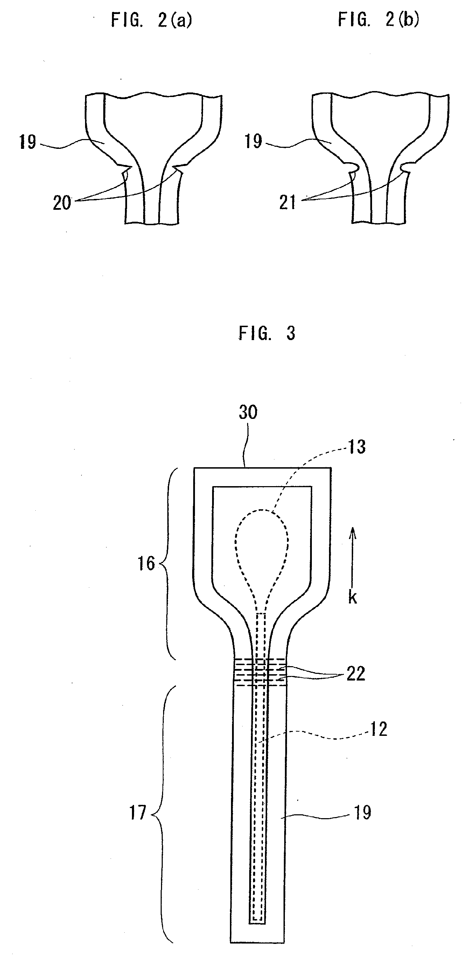

Figs. 2(a) and 2(b) show other notch parts having different shapes. As shown in Figs. 2(a) and 2(b), notch parts 20 and 21 that are cut in V-shape and U-shape, respectively may be employed.

As another means for separating the impregnated part cover region 16 from the shank cover region 17, there is for example a plurality of perforated lines 22 that are formed in the width direction of the gas impervious film 30, as shown in Fig. 3. The gas impervious film 30 of Fig. 3 is obtained by laminating and integrating, as shown in Fig. 4, a film for thermal bond 24 and heat resistance film 25 in this order on the surface of a gas impervious film 26, such as metallic foil, the underside of which is laminated with a film for thermal bond 23. Then, the plurality of perforated lines 22 are formed in the heat resistance film 25 in the surface of the gas impervious film 30. By drawing the impregnated part cover region 16 at the time of unpacking (the direction of drawing is indicated by arrow "k" in Fig. 3), the gas impervious film 15 cleaves at the region where the perforated lines 22 are formed, and the application liquid impregnated part 13 is exposed. Also in this case, the perforated lines 22 are preferably formed in trapezoid such that the end part of the shank cover region 17 separated from the impregnated part cover region 16 is shaped so as to be chamfered. If it is difficult to do so, linear perforated lines 22 are preferably formed away from the application liquid impregnated part 13, as shown in Fig. 3.

Application liquid to be impregnated in the application liquid impregnated part 13 is properly selected depending on the use and purpose. Examples include medical liquids; makeup liquids; and cleaning liquids of machine parts, electronic parts, etc. It is also possible to impregnate organic solvents or other cleaning liquids for removing contamination in human body and clothing, water, etc.

Examples of the medical liquids are medical liquids used for disinfection, sterilization or medical treatment. There are for example disinfectants such as acrinol, alkyldiaminoethylglycine HCl, ethanol, hydrogen peroxide, glutaral, cresol, chlorhexidine gluconate, sodium hypochlorite, benzalkonium chloride, benzethonium chloride, povidone-iodine (Isodine Solution, etc.), mercurochrome and iodine tincture. As long as these medical liquids are liquid, they may directly be impregnated in the application liquid impregnated part 13. Alternatively, they may be dissolved in a solvent such as water and then impregnated in the application liquid impregnated part 13.

Examples of the makeup liquids are plant extracts such as aloe extract, collagen, lotion, baby oil, baby lotion, mugwort extract, luffa extract and rice sugar extract, and various aromatics. Besides, nail-polish remover and cleansing liquid may be used. As long as these makeup liquids are liquid, they may directly be impregnated in the application liquid impregnated part 13. Alternatively, they may be dissolved in a solvent such as water and then impregnated in the application liquid impregnated part 13.

Examples of the cleaning liquids are organic solvents such as acetone and alcohols, and machine oils.

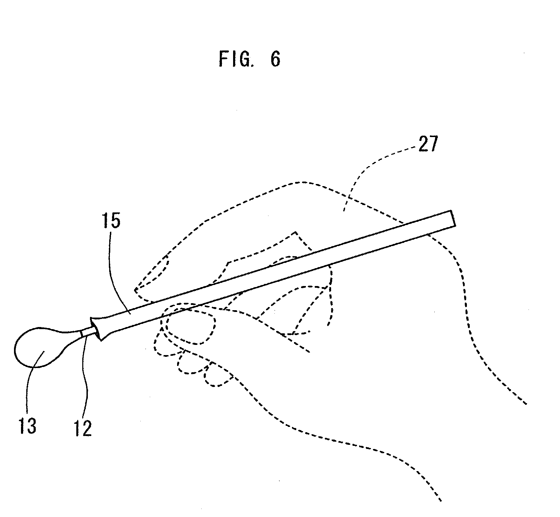

The following is the use of the applicator 11 by taking Figs. 5(a) and 5(b) as example. As shown in Fig. 5(a), the gas impervious film 15 is drawn along the notch parts 18 and the impregnated part cover region 16 is separated and removed from the shank cover region 17, thereby exposing the application liquid impregnated part 13. Then, as shown in Fig. 6, in the state that the shank 12 is covered with the gas impervious film 15 of the shank cover region 17, application is performed while gripping the shank 12 by a hand 27. This manner avoids that the hand 27 becomes dirty during use, even if during the time that the applicator is stored or transported, the application liquid leaks from the application liquid impregnated part 13 and adheres on the surface of the shank 12.

Further, the remaining end part of the impervious film 15 has a chamfered shape that slants downwardly to its edge part. This prevents a sharp point of the gas impervious film 15 from erroneously abutting the affected part at the time of application. Note that if the film edge part of the shank cover region 17 is sufficiently apart from the application liquid impregnated part 13, such a chamfered shape is not necessarily required. In the case of the V-shape and U-shape notch parts 20 and 21 shown in Figs. 2(a) and 2(b), respectively, the edge part of the gas impervious film 15 so separated is always chamfered and therefore preferable.

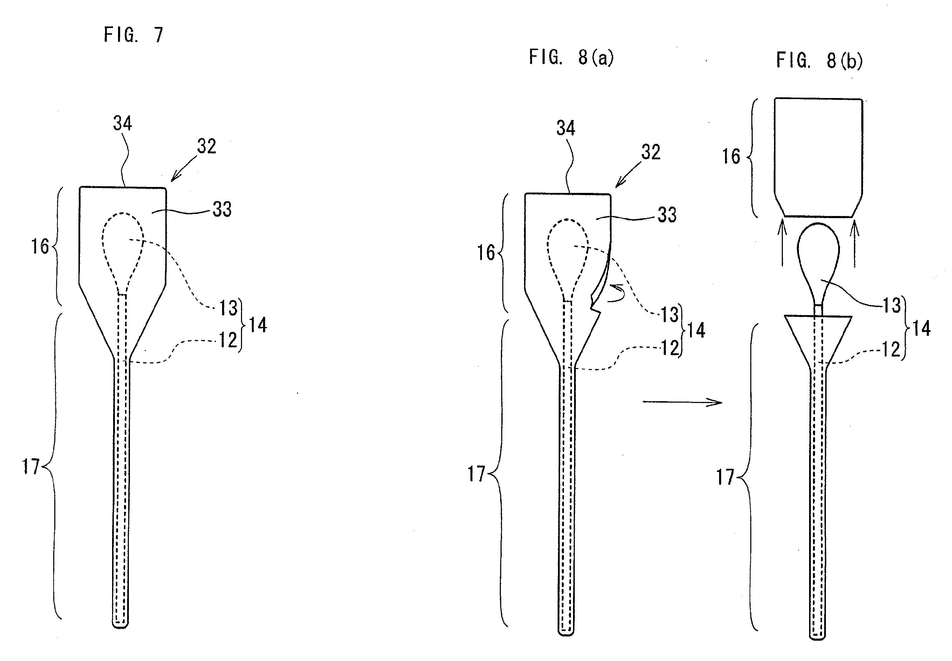

Another preferred embodiment of the present invention is shown in Fig. 7. An applicator 32 is obtained by wrapping a swab 14 in a film for thermal bond 33 and performing heat sealing at the same time that a peripheral part 34 is cut with a heated knife, etc. Since the applicator 32 is obtained only by wrapping the swab 14 in the film for thermal bond 33, this is suitable particularly when the application liquid impregnated in the application liquid-impregnated part 13 is a low volatile liquid such as oil. It is preferable that the film for thermal bond 33 is made of a uniaxial oriented film and the direction of its orientation is orthogonal to the shank 12. Thereby, at the time of use, the vicinity of the boundary between the impregnated part cover region 16 and shank cover region 17 can easily be cut away by hand to remove the impregnated part cover region 16. If required, the film for thermal bond 33 may be provided with a sealing part for separating the impregnated part cover region 16. Alternatively, it may be separated with the use of scissors, etc. Otherwise, this preferred embodiment is identical with the foregoing embodiment. Accordingly, like parts have similar reference numbers and their description are omitted here.

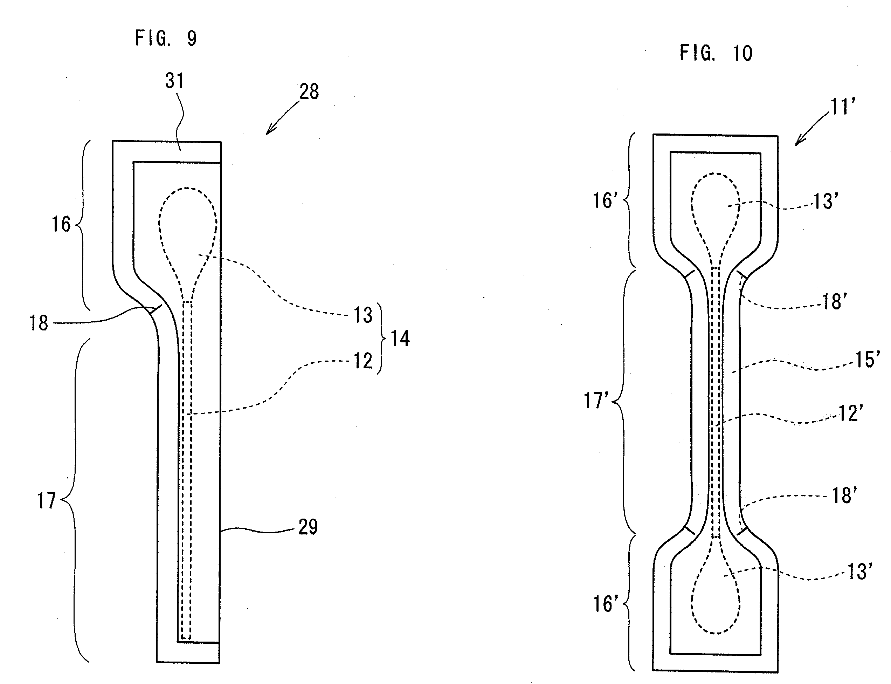

A further preferred embodiment of the present invention is shown in Fig. 9. An applicator 28 is obtained by folding a gas impervious film 29 sandwiching an applicator body 14 therebetween and then sealing an overlapped peripheral part 31 (sealing part) by thermal bond or the like. Otherwise, this preferred embodiment is identical with the foregoing embodiment. Accordingly, like parts have similar reference numbers and their description are omitted here.

In the foregoing preferred embodiments, the application liquid impregnated part 13 is formed on one end of the shank 12. In an alternative, application liquid impregnated parts 13' and 13' may be formed on both ends of a shank 12', as shown in Fig. 10. In this case, a narrow shank cover region 17' is formed in a middle part of a gas impervious film 15', regions 16' and 16' covering the application liquid impregnated parts 13' are formed on the both ends of the shank 12', and notch parts 18' are formed on the boundaries between the region 17' and the both regions 16', 16'. Otherwise, the construction is identical with the foregoing preferred embodiments.

As required, the shank 12 (12') may be adhered on the internal surface of the shank cover region 17 (17') of the gas impervious film.

According to the present invention, an application liquid such as a medical liquid is previously impregnated in the application liquid-impregnated part and sealingly packed with the gas impervious film. It is therefore convenient to use the applicator immediately after unpacking the gas impervious film. In addition, it is avoidable that the user's hand becomes dirty with an application liquid such as a medical liquid by separating only the region covering the application liquid impregnated part and then gripping the shank via the film covering the shank.

## Claims

1. An applicator comprising:
a shank,
an application liquid-impregnated part provided on at least one end of said shank; and
a film covering said shank and said application liquid-impregnated part,
wherein said film comprises a shank cover region and an impregnated part cover region that is formed integrally with and separable from said shank cover region, and the width of said shank cover region of said film is such that said shank can be gripped when it is covered with said film.

2. The applicator according to claim 1 wherein said film is a gas impervious film.

3. The applicator according to claim 2 wherein said gas impervious film is a film obtained by laminating a film for thermal bond on a metallic foil.

4. The applicator according to claim 2 or claim 3 wherein said gas impervious film is provided with a sealing part for sealingly packing said impregnated part cover region and said shank cover region.

5. The applicator according to claim 4 wherein said sealing part is provided with means for separating said impregnated part cover region and said shank cover region.

6. The applicator according to claim 5 wherein said means is a notch part.

7. The applicator according to any of claims 1 to 6 wherein said film is subjected to heat sealing at the same time it is cut.

8. The applicator according to any of claims 1 to 7 wherein said application liquid impregnated part is a foam body or fiber aggregate that has liquid absorbing property.

9. The applicator according to any of claims 1 to 8 wherein said application liquid is medical liquid, makeup liquid or cleaning liquid.

10. An application method using the applicator according to any of claims 1 to 9, which comprises steps of exposing said application liquid-impregnated part by separating said impregnated part cover region from said shank cover region in said film, and then performing application while gripping said shank covered with said film.
